# EUROPEAN PATENT SPECIFICATION

(11) **EP 3 634 463 B1**
(45) Date of publication and mention of the grant of the patent: **27.09.2023**
(21) Application number: 18798594.0
(22) Date of filing: 11.05.2018
(51) Int. Cl.: A61K 38/13, A61P 13/12, C07K 7/64, G01N 33/48, A61K 31/365, A61K 31/573

(54) **IMPROVED PROTOCOL FOR TREATMENT OF LUPUS NEPHRITIS**
VERBESSERTES PROTOKOLL ZUR BEHANDLUNG VON LUPUS NEPHRITIS
PROTOCOLE AMÉLIORÉ POUR LE TRAITEMENT DE LA NÉPHROPATHIE LUPIQUE

(30) Priority: 12.05.2017 US 201762505734 P; 04.08.2017 US 201762541612 P; 07.12.2017 US 201715835219
(43) Date of publication of application: 15.04.2020
(60) Divisional application: 23181300.7
(73) Proprietor: Aurinia Pharmaceuticals Inc., Victoria, BC V8Z 7X8 (CA)
(72) Inventor: SOLOMONS, Neil, Victoria, British Columbia V8Z 7X8 (CA); HUIZINGA, Robert B, Victoria, British Columbia V8Z 7X8 (CA)
(74) Representative: V.O.
(86) International application number: PCT/IB2018/000828
(87) International publication number: WO 2018/207026

(56) References cited:
- YAHYA, R. et al.: "AURION Study: 12 week data of multi-target therapy with voclosporin, MMF and steroids for lupus nephritis", Clinical and Experimental Rheumatology, vol. 34, no. Issue Number 4, Suppl. 99, 5 October 2016 (2016-10-05), page S-15, XP009519458,
- HUIZINGA, R.B. et al.: "AURION Study: 48-week data of multi-target therapy with voclosporin, MMF and steroids for lupus nephritis", The 12th International Congress on Systemic Lupus Erythematosus (LUPUS 2017) & the 7th Asian Congress on Autoimmunity (ACA 2017, 27 March 2017 (2017-03-27), XP055677571, Retrieved from the Internet: URL:http://c.eqcdn.com/349da054eda96a4b678 1a0902e2929ab/auriniapharma/db/246/910/pdf /LUPUS+2017+PresentationFINAL.pdf [retrieved on 2018-11-19]
- AURINIA PHARMACEUTICALS: "Aurinia highlights 48-week data from open-label AURION study at 12th International Congress on SLE (LUPUS 2017) & the 7th Asian Congress on Autoimmunity (ACA 2017", Press Release, 27 March 2017 (2017-03-27), XP055677565, Retrieved from the Internet: URL:https://ir.auriniapharma.com/press-rel eases/detail/81 [retrieved on 2018-11-19]
- PENDERGRAFT, W.F.: "AURA-LV: Successful treatment of active lupus nephritis with voclosporin", Journal of the American Society of Nephrology, vol. 27, November 2016 (2016-11), page 2B, XP055677563,
- AURINIA PHARMACEUTICALS: "Aurinia releases additional 48-week data from the AURA-LV Study during late-breaking session at the National Kidney Foundation 2017 Spring Clinical Meetings", Press Release, 20 April 2017 (2017-04-20), XP055677560, Retrieved from the Internet: URL:https://ir.auriniapharma.com/press-rel eases/detail/85/aurinia-releases-additiona l-48-week-data-from-the-aura-lv [retrieved on 2016-11-19]

## Description

### TECHNICAL FIELD

The invention relates to treatment of lupus nephritis and other proteinuric kidney diseases with voclosorin. More specifically it relates to pharmacodynamic dosing of subjects in accordance with an improved protocol for treatment.

### BACKGROUND ART

Lupus nephritis (LN) one of a number of proteinuric kidney diseases wherein an inflammation of the kidneys is caused by systemic lupus erythematosus (SLE) whereby up to 60% of SLE patients develop LN. LN is a debilitating and costly disease often leading to renal failure which requires dialysis, or renal transplant and often results in death. Indeed, patients with renal failure have an over 60-fold increased risk of premature death compared to SLE patients in general. A clinical sign of LN is leakage of blood proteins into the urine and the disease can be diagnosed by a number of factors, including urinary protein/creatinine ratio (UPCR) wherein a UPCR of greater than 0.5mg/mg is indicative of the condition being in an active state. Further, certain markers in the blood can also be diagnostic--for example, complement 3 (C3), complement 4 (C4) and anti-dsDNA antibodies.

The standard of care for LN has not met with a great deal of success. The standard of care is use of mycophenolate mofetil (MMF) or intravenous cyclophosphamide. With these treatments partial remission was found only in approximately 50% of cases and complete remission was shown in less than 10% of the subjects. Thus, there is clearly a need for a treatment that improves these outcomes.

Voclosporin is an analog of cyclosporin A that has been found useful for treating autoimmune diseases and as an immunosuppressant in organ transplantation.

Mixtures of the E and Z isomers of voclosporin are described in U.S. Patent 6,998,385. Mixtures with a preponderance of the E-isomer are described in U.S. Patent 7,332,472. The '472 patent describes a number of indications which can be treated with the isomeric voclosporin mixture including glomerulonephritis. However, although some animal studies are described, no protocols in humans are disclosed.

Various formulations of voclosporin mixtures are also described in U.S. Patent Nos. 7,060,672; 7,429,562 and 7,829,533.

In October 2016, the results of a clinical study conducted on behalf of Aurinia Pharmaceuticals were published as an abstract. According to the abstract, the subjects, who were afflicted with lupus nephritis (LN), were dosed with 23.7mg of voclosporin twice daily in combination with mycophenolate mofetil (MMF) and reducing cortical steroid dose over 24 weeks for which data were presented. Entry criteria for the study included determination of a urine protein creatinine ratio (UPCR) of >1.0mg/mg or >1.5mg/mg depending on the classification of a renal biopsy and an eGFR (estimated glomerular filtration rate) of >45mol/mn/1.73m² as well as serologic evidence of LN. The results of this protocol showed complete remission or partial remission in a large percentage of subjects.

In addition, it was shown that subjects who achieved a >25% reduction in UPCR at 8 weeks were likely to maintain benefit throughout the 24-week or 48 week protocol.

In a news release sent by Aurinia Pharmaceuticals on 1 March 2017, the results of more extensive clinical study involving mycophenolate mofetil (MMF) and reducing corticosterone dosages, but using in addition to 23.7mg of voclosporin twice daily, a higher dose of 39.5mg twice daily were described. The results of this study showed successful complete or partial remission in a large number of patients at 24 weeks and 48 weeks. It appeared that the lower dosage of 23.7mg twice daily (BID) was even more effective than the higher dosage of 39.5mg twice daily (BID).

Data regarding predictability of success with respect to complete remission (CR) based on various criteria measured after 8 weeks of treatment with 23.7 bid of Voclosporin along with MMF-1 and steroid taper were presented by the present inventors at the 12^{th} International Congress on SLE on 27 March 2017. These criteria included UPCR (less than 25% reduction considered to show ineffectiveness) as well as normalization of complements 3 and 4 (C3 and C4) and of anti-dsDNA. However, the criteria for normalization of C3, C4 and anti-dsDNA were not disclosed.

An earlier study with respect to lupus treatment with cyclophosphamide rather than Voclosporin suggested normalization of C4 as a marker. Dall'Era, M. et al Arth. Care and Res. (2011) 63:351-357.

It has now been found that successful results, including a diminution in the number and severity of side effects can be obtained by altering the protocols disclosed in these publications by providing a pharmacodynamic dosing schedule based on individual patient responses. In addition, it has been found that even lower dosages of voclosporin-*i.e*., 15.8mg of voclosporin twice daily or 7.9mg of voclosporin twice daily are effective.

### DISCLOSURE OF THE INVENTION

The present invention, thus, provides an improved protocol for treatment of lupus nephritis and other proteinuric kidney diseases that takes advantage of assessments of parameters associated with the response of individual subjects. The invention is a personalized form of a protocol for treatment of proteinuric kidney diseases including protocols that employ low dosage of voclosporin. The voclosporin used is preferably a mixture of greater than about 80% E isomer and less than about 20% Z isomer, and more preferably greater than about 90% E isomer and less than about 10% Z isomer. The protocol employs daily dosages of voclosporin over a projected period of 24, 48, 52 weeks or longer wherein the voclosporin is administered twice daily (BID). Suitable dosages are in increments of 7.9 mg including 39.5 mg, 31.6 mg, 23.7 mg, 15.8 mg or 7.9 mg. Low dosages show superior results compared to a higher dose of 39.5 mg each of such administrations carried out twice daily. Doses as low as 15.8 mg or 7.9 mg twice daily are effective. The protocol preferably further includes administering to the subject an effective amount of MMF and/or an effective amount of a corticosteroid, typically prednisone, in a reducing dosage level across the time period of the study.

While it has been verified that the protocols of the invention are effective for lupus nephritis, such results indicate the same protocols can be used to generally treat proteinuric kidney diseases. Proteinuric kidney diseases that can be treated include: Diabetic nephropathy, nephrotic syndromes (i.e. intrinsic renal failure), nephritic syndromes, toxic lesions of kidneys, glomerular diseases, such as membranous glomerulonephritis, focal segmental glomerulosclerosis (FSGS), IgA nephropathy (i.e., Berger's disease), IgM nephropathy, membranoproliferative glomerulonephritis, membranous nephropathy, minimal change disease, hypertensive nephrosclerosis and interstitial nephritis.

One of the side effects of treatment with voclosporin is an unwanted decrease in the estimated glomerular filtration rate (eGFR). One invention protocol is designed to reduce the incidence of this undesirable side effect by adjusting the dosage in accordance with the response of the subject.

Thus, in one aspect, the invention is directed to a predetermined daily dosage of effective amounts of voclosporin over a projected treatment period of at least 24 weeks for use in a pharmacodynamic method to treat a proteinuric kidney disease, wherein:
(a) the estimated Glomerular Filtration Rate (eGFR) of said subject is assssed at at least a first time point and a second time point on different days of said treatment period, and
(b)
   (i) if the eGFR of said subject decreases by more than a target % to below a predetermined value between said first and second time points, the daily dosage is reduced by increment(s) of 7.9mg BID or the administering of voclosporin to said subject is stopped;
   (ii) if the eGFR of said subject decreases between said first and second time points by more than a target % in the range of 20-45%, to below a predetermined value in the range of 50-90 ml/min/1.73m², administering the same predetermined daily dosage of voclosporin to said subject is continued.

In one specific embodiment, the invention includes use of a predetermined daily dosage of effective amounts of voclosporin over a projected treatment period of at least 24 weeks in a pharmacodynamic method to treat lupus nephritis, which is administered to a subject diagnosed with lupus nephritis, wherein:
(a) the glomerular filtration rate (eGFR) of said subject is assessed at at least a first time point and a second time point on different days of said treatment period, and
(b)
   (i) if the eGFR of said subject decreases by >30% to a value of below 60 mL/min/1.73m² between said first and second time points, the administering of voclosporin to said subject is stopped;
   (ii) if the eGFR of said subject decreases by between 20% to 30% to a value of below 60 ml/min/1.73m² between said first and second time points, a reduced dosage of voclosporin is administered to said subject;
   (iii) if the eGFR of said subject decreases by <20% between said first and second time points, administering the same predetermined daily dosage of voclosporin to said subject is continued.

The eGFR of 60 mL/min/1.73m² noted above is typically used; however, higher values such as 90 mL/min/1.73m² or 75 or 70 mL/min/1.73m² or lower value such as 50 mL/min/1.73m² or 55 mL/min/1.73m² could also be used.

The pharmacodynamic method may employ a third time point subsequent to the first and second time point wherein the target percentage reduction is again determined and if the percentage reduction as compared to the first time point is less than the target percentage, treatment may be restored, or if the percentage decrease is greater than the target percentage exhibited at the second time point, further reduction in dosage may be indicated.

References to the methods of treatment by therapy in the present specification have to be interpreted as reference to compounds, i.e., voclosporin, or compositions of the present invention for use in those methods.

It has also been found that the effectiveness of the protocol can be evaluated after only a portion of the treatment period has elapsed. This can be done in lieu of, or in addition to, the foregoing protocol. Therefore, because it is generally undesirable to continue an ineffective treatment an assessment at a time point earlier in the protocol than the end point planned is conducted and if the effectiveness of treatment is not confirmed, the treatment is terminated. In the present case, such termination may be helpful since generally administration of an immunosuppressant is not recommended unless some benefit is achieved.

For example, the assessment may include determining the UPCR at first and second time points early in the protocol wherein the first point is determined at the outset of the protocol and stopping the administration of voclosporin to the subject if the UPCR has not been reduced by a predetermined amount, for example by 15% or 20% or 25% or 35% at the second time point. The UPCR can be determined by any standard technique, e.g., using first morning void or a 24 hour urine sample. This evaluation may be supplemented or substituted by evaluation of the concentration of C3 and C4 in the blood. Failure of the treatment to result in normalizing C3/C4 concentration indicates lack of success. The rule for stopping treatment if a combination of these factors is employed is that treatment will be stopped if neither criterion for success is met - i.e. the subject shows no satisfactory reduction in UPCR at the second time point and no normalization of C3/C4 at the second time point. In the alternative, either criterion could be used alone.

Alternatively, if it appears that the subject is in complete remission, it may be unnecessary to continue treatment.

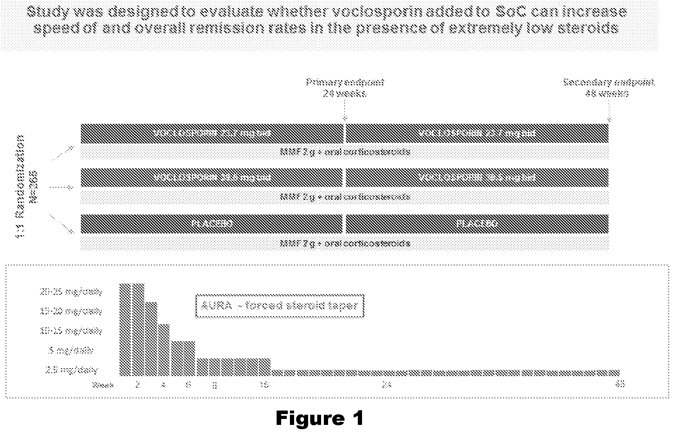

In any of the protocols above, preferably, a dosage of MMF and a reducing dosage of corticosteroid is also administered during the treatment period. Typically, MMF is administered at the level of 2 grams daily and oral corticosteroids are administered in daily dosages diminishing from 20-25mg daily to 2.5mg daily over a period of 16 weeks. The reduced dosages then continue throughout the study. These protocols are shown in Figure 1.

In all cases, subjects who would be amenable to this treatment are identified by screening said subject prior to conducting said method on said subject by:
(a) determining that the urine protein creatinine ratio (UPCR) of said subject is >1.5mg/mg or >1mg/mg depending on renal biopsy as preferably measured by first morning void; and
(b) determining said subject has an eGFR as measured by the Chronic Kidney Disease Epidemiology Collaboration equation (CKD-EPI) of >45 mL/min/1.73m²or any other suitable method such as the Modification of Diet in Renal Disease (MDRD) Study equation. If the determinations of subparagraphs (a) and (b) are positive, the subject is considered suitable for subjection to the protocol.

In addition, lowered levels of voclosporin dosage have been shown to be effective.

Thus, in another aspect, the invention is directed to a method to treat lupus nephritis which method comprises administering to a subject diagnosed with lupus nephritis a predetermined daily dosage of effective amounts of voclosporin wherein said effective amount is either 15.8mg BID or 7.9mg BID. Surprisingly, it has been found that these lower dosages are effective in a majority of patients, especially if the treatment is prolonged beyond 24 weeks. A dosage of 31.6 mg BID (4 capsules) could also be employed. Pharmacodynamic dosing can be applied in these instances as well.

It is also advantageous and part of the invention to evaluate a subject who has been treated with the protocol at the end of the treatment period to determine whether a complete or partial remission has occurred. Further evaluations are included at a time subsequent to termination of the treatment to assess whether the remission achieved according to the measurement at the end of the treatment is being maintained. Such evaluation may also be done at intermediate times during treatment to determine whether dosage can be reduced. In an exemplary embodiment used simply for illustration a dosage of 23.6 mg voclosporin BID may be reduced to 15.8 mg or 7.9 mg BID based on such results.

The evaluation for effectiveness can be based on the protein/creatinine ratio in urine (UPCR) where a ratio of ≤ 0.5 mg/mg indicates complete response; alternatively, or in addition, an eGFR of ≥ 60 mL/min/1.73m² or no decrease from baseline and eGFR of ≥ 20% is shown. Other indications of complete response include lack of need for rescue medications such as intravenous steroids, cyclophosphamide or a need for ≤ 10 mg prednisone for more than three consecutive days or more than seven days total. These evaluations may be performed at any point in the treatment.

### BRIEF DESCRIPTION OF THE DRAWINGS

Figure 1 shows graphically the design of a protocol for treatment of lupus nephritis with voclosporin upon which the pharmacodynamic treatment of the invention is superimposed.
Figure 2 is a bar graph showing a comparison of complete remission (CR) at 48 weeks and partial remission (PR) at 48 weeks for low dose as compared to high dose of voclosporin (VCS). The PR percentages include the CR percentages.

### MODES OF CARRYING OUT THE INVENTION

As noted above, the pharmacodynamic protocol of the invention relates to adjustment of administration of voclosporin depending on certain physiological indicators of the subjects. As further noted, the voclosporin administration is preferably conducted with a background of administration of MMF and of corticosteroids.

In general, the treatment protocols on which the invention is based show dramatically better results than the current standard of care-the rate of complete remission is almost 50% as compared to much lower levels achieved using the current standard of care treatment and the rate of partial remission after 48 weeks is even higher, wherein this rate (PR) includes the subjects with complete remission (CR). As shown below, a lower dosage of voclosporin is more effective than a higher dose in a comparable protocol wherein complete remission was obtained in 40% of the subjects at the higher dosage as opposed to 49% of the subjects in the lower dose group.

Critical to the present invention is pharmacodynamic administration of the voclosporin component. This pharmacodynamic administration is essential as the effects of this drug may be too intense and/or have undesirable side effects or the results may indicate lack of effectiveness and thus the dosage is reduced or interrupted to permit homeostasis to reestablish a suitable set of physiological parameters.

The protocols are designed to cover treatment periods of at least 24 weeks and may extend for longer periods of time, for example 48 weeks or 52 weeks. The regimens involve daily dosages of the voclosporin component, typically twice daily, although alternative frequencies could be employed, such as once a day, three times a day or four times a day based on reactions of the patient and convenience. As an illustration, the protocol will be described below in terms of a 23.7mg twice daily (BID) dosage; of course if the voclosporin preparation is administered four times daily, the dosage would be cut in half for each administration and if the administration were only once per day, the dosage for the once a day administration would be double the 23.7mg administered BID.

In addition, as to the pharmacodynamic protocols of the invention, the dosages may be any combination of the 7.9 mg basic units, and thus can be 7.9 mg, 15.8 mg, 31.6 mg, or 39.5 mg, as well as the exemplified 23.7 mg.

The dosage indicated, for example 23.7mg, (or any other specified dose) is subject to slight variations, typically ±10% or, alternatively, for 23.7mg specified between 21mg and 26mg BID. This is due to inconsistencies in pharmaceutical manufacture and the ideal dosage is the specified dose-*i.e.*, for example, 23.7mg BID. Comparable variations applied to the alternative dosages, and to the differential adjustment.

### Adjustment Based on eGFR:

One critical parameter used to assess the desirability of dosage reduction is the eGFR using the CKD-EP1 formula or other appropriate method. Chronic kidney disease is defined as eGFR as <60mL/min/1.73m² for >three months with or without kidney damage. As noted above, a further decrease in eGFR is a negative side effect that may occur during treatment. If the decrease is too severe, the protocol should be altered in accordance with the prescription of the invention. Typically, a baseline value of the eGFR is established either at the beginning of the protocol or at some "first time point" during the protocol. If the decrease is greater than a target percentage, which is typically between 20%-45% as compared to the first time point, a reduction in dosage is indicated, including a reduction to zero or stopping treatment. If the decrease is less than that target percentage maintenance of treatment at the same level as indicated. In addition to an indication that treatment should be reduced or terminated based on the eGFR reduction, reduction below a certain value is also indicative of a need to modify the treatment. This predetermined value is typically in the range of 50-90 mL/min/1.73m².

As stated above, a baseline value for eGFR is established at the outset of the treatment or during treatment. This is typically done on the first day of the treatment before any administration of the drugs in the protocol. This baseline is used as a criterion for adjusting dosage. However, a first time point could be established at any arbitrarily selected time during the protocol.

In one exemplary protocol, at a second time point subsequent to the first which can be on any day of the treatment, a subject with >30% decrease in eGFR from the baseline to <60ml/min/1.73m² (or, in some cases, a higher cut off) should have the treatment interrupted until a repeat test can be performed, but if the decrease is confirmed and not due to contributing factors (such as a high baseline eGFR, the addition or modification of non-steroidal anti-inflammatory drugs, angiotensin converting enzyme inhibitors, angiotensin 2 inhibitor blockers, or a concurrent state of dehydration, etc.), the treatment should be withheld until a third time point determination typically within 48 hours. If the >30% decrease is not maintained, treatment is restored to two-thirds or one-third of the original dosage and increased as tolerated to the 23.7mg level BID.

For convenience, the 23.7mg administration is administered orally in the form of three capsules containing 7.9mg each. Thus, it is easy to provide two-thirds of the standard dosage by administering at any given time only two of the three capsules.

With regard to the second time point, in this exemplary protocol a subject having a decrease of >20% in eGFR to <60mL/min/1.73m² but a decrease of less than 30% reduction as compared to baseline, the treatment is not interrupted but the dosage is reduced. Reduction in increments of 7.9mg is preferred. Again, assessment at an additional time point at any subsequent day during the treatment showing baseline values are restored indicates that the original dosage level of 23.7mg BID can be resumed.

### Adjustment Based on UPCR Reduction and/or C3/C4 Normalization

Suitable criteria for early evaluation of the probability of success - i.e. complete or partial remission by the end of a planned protocol, have been identified which permit discontinuation of the treatment earlier than the end point if it is highly unlikely that the subject will benefit from continuing on the dosage schedule. These criteria are reduction in UPCR and normalization of C3/C4. These can be used in combination or in the alternative. It has been found that a determination early in a 24-week or 48 week protocol of probable success can be used to determine whether treatment with voclosporin should continue; such findings are applicable to protocols of any substantial length. By way of illustration, if the UPCR has not been reduced by a satisfactory amount early in the regimen or if C3/C4 has not been normalized early in the regimen, one might conclude that the chances of improvement over an extended treatment period are diminished. Application of these criteria in an exemplary trial is shown in detail in Example 3.

However, a rule for stopping treatment is based either on UPCR reduction or C3/C4 normalization taken alone or in combination. If the combination is used, then failure with respect to both criteria would dictate termination of treatment. Decision would be based on the values of the sensitivity, specificity and positive and negative predictive values shown in the tables set forth in Example 3. These terms are defined in the Example.

As an illustration only, such determination can be made at approximately 8 weeks subsequent to the beginning of the regimen; a time frame of 6-10 weeks could be employed for the approximation of 8 weeks. If a decrease of, for example, >25% of UPCR is not achieved, it appears unlikely the subject will benefit from further treatment and the protocol is stopped. As shown in the Example, this can be supplemented with evaluation of C3/C4 normalization - if this is done, it is desirable that both determinations be made at the same early time point.

### Auxiliary Therapeutics

The voclosporin treatment of the invention is supplemented with MMF and reduced amounts of corticosteroids.

For example, with respect to corticosteroids, subjects who weigh 45kg or more may receive 0.5 grams of methylprednisolone on days 1 and 2 of the study intravenously and then beginning on day 3, oral corticosteroid therapy. Subjects weighing <45kg receive only half these dosages.

For oral prednisone, the starting dosage for oral administration is 20mg/day for subjects <45kg and 25mg/day for subjects who weigh >45kg. The dosage is reduced according to the protocol shown in Table 1.

**Table 1 Dosing Schedule for IV Methylprednisolone and Daily Oral Prednisone (mg)**

| | **Subjects <45 kg** | **Subjects ≥45 kg** | **In Case of Prior IV Steroids During Screening (Pre-randomization)** |
|---|---|---|---|
| Weeks 1-2⁽¹⁾ | | | |
| Days 1-2⁽²⁾ | 0.25 g (IV) | 0.5 g (IV) | 1 g minus prior IV steroids mg or (0.5 g minus prior IV steroids mg for subjects who weigh <45 kg)⁽³⁾ |
| Days 3-13 | 20 mg (oral) | 25 mg (oral) | |
| Week 2 (Day 14) | 15 mg (oral) | 20 mg (oral) | |
| Week 4 (Day 28) | 10 mg (oral) | 15 mg (oral) | |
| Week 6 (Day 42)⁽⁴⁾ | 10 mg (oral) | 10 mg (oral) | |
| Week 8 (Day 56) | 5 mg (oral) | 5 mg (oral) | |
| Week 12 (Day 84) | 5 mg (oral) | 5 mg (oral) | |
| Week 16 (Day 112) | 2.5 mg (oral) | 2.5 mg (oral) | |

| | | | |
|---|---|---|---|
| 1 Day 0-13: Oral steroids dosed according to subject weight and then tapered beginning at Day 14. 2 Oral corticosteroids may be commenced on Days 1 or 2 if corticosteroids are administered during screening. 3 It is recognized that dosing with IV methylprednisolone as described in Section 7.2.2.2, Corticosteroids may not be in the subject's best interest if they have already received therapy within the 3 months prior to screening. In this case, the Investigator may be permitted to omit the administration of further IV methylprednisolone but only after discussion with the Medical Monitor. 4 Week 6 is not a scheduled study visit, a phone call can be performed to decide further tapering for subjects. Notes: Oral prednisone taper should be done within ±3 days of specified timeframe. When clinically indicated, subjects are allowed to be completely titrated off of oral corticosteroids. Abbreviation: IV = Intravenous. | | | |

In contrast to the reducing dose of corticosteroid, the same dose of MMF is maintained throughout the study at a twice daily dosage before meals with a glass of water. Typically, each individual dose is one gram resulting in a total dosage of two grams per day although in the alternative, the subject may be administered 500mg four times daily.

### Low Dosage

In addition to the above-described pharmacodynamic protocols, applicants have found that a substantially lower dosage than expected is effective in large numbers of subjects such that voclosporin for use in a method to treat lupus nephritis in subjects either with or without pharmacodynamic adjustments can be based on a dosage level of either 15.8mg BID or 7.9mg BID. The increments of 7.9mg are dictated by the availability of capsules containing the 7.9mg dosage level which provides a convenient platform for dosage alterations.

One additional aspect of the invention is therefore voclosporin for use in a method to treat lupus nephritis wherein either a logically adjusted dosage or a constant dosage of either 15.8mg BID or 7.9mg BID is employed. In these protocols, as would be the case for higher dosages of voclosporin, the above described background administration of MMF and corticosteroids is included in the protocol.

### General Factors

In all cases, the subjects are evaluated for success of the treatment both on the completion of the treatment and at extended periods thereafter. Typical treatment periods are at least 24 weeks, but preferably 48 weeks or more. Reevaluation after the termination of the treatment period over a period of 1-2 weeks or longer is also employed. Subjects are evaluated for complete or partial remission. Complete remission (CR) is defined as: Confirmed protein/creatinine ratio of ≤0.5 mg/mg, and eGFR ≥60 mL/min/1.73 m² or no confirmed decrease from baseline in eGFR of ≥20%. Partial remission is defined as: 50% reduction in UPCR from baseline.

By establishing a pharmacodynamic dosing regimen, the effectiveness of the protocol in treatment of lupus nephritis can be maximized while minimizing undesirable side effects.

The length of the treatment protocol in all cases will vary from at least 8 weeks to for example 12, 16, 24 and 48 weeks or 52 weeks or even longer, up to 60 weeks including stopping points between the levels mentioned. For example, a treatment protocol of 10 or 11 or 15 or 20 or 29 or 31 or 36 or 43 or 51 or 55 weeks could be employed and is within the scope of the invention. The evaluation described above is conducted at the end of the protocol as well as at a suitable time period or multiple time periods thereafter. These time periods are generally 1-2 weeks to 4-5 months subsequent to terminating the dosage and intervening at time intervals are included within the scope of the invention as well.

This statement regarding time intervals applies to the invention pharmacodynamic treatment protocols, regardless of base dosage levels.

The following examples are to illustrate, not to limit the invention.

### Example 1

### 48 Week Study of LN Treatment

The subjects enrolled in the study were divided into three groups, 88 subjects are in a control group who were administered 2g MMF daily as well as oral corticosteroids-*i.e.*, prednisone in a tapering dosage shown graphically in Figure 1- beginning at 20-25mg daily reduced gradually after the 12^{th} week to 2.5mg daily. (Some subjects received lower doses of MMF due to gastrointestinal problems). 89 subjects in the low dosage group received this background treatment, but in addition were administered three capsules containing 7.9mg (*i.e.* 23.7mg) of voclosporin each twice daily. The voclosporin used in this study comprised greater than 90% E isomer. A third group which was comprised of 88 subjects received a similar background treatment but in addition were dosed with five 7.9mg capsules *i.e.* 39.5mg twice daily. The study was conducted over a period of 48 weeks and safety was evaluated at 24 weeks.

Subjects were screened prior to admission to the study by (a) determining that the urine protein creatinine ratio (UPCR) as >1.5mg/mg as measured by first morning void, and (b) that the eGFR as measured by Chronic Kidney Disease Epidemiology Collaboration equation (CKD-EP1) of >45 ml/min/1.73m². Subjects were assessed after 24 weeks and 48 weeks as well as a subsequent evaluation at 50 weeks.

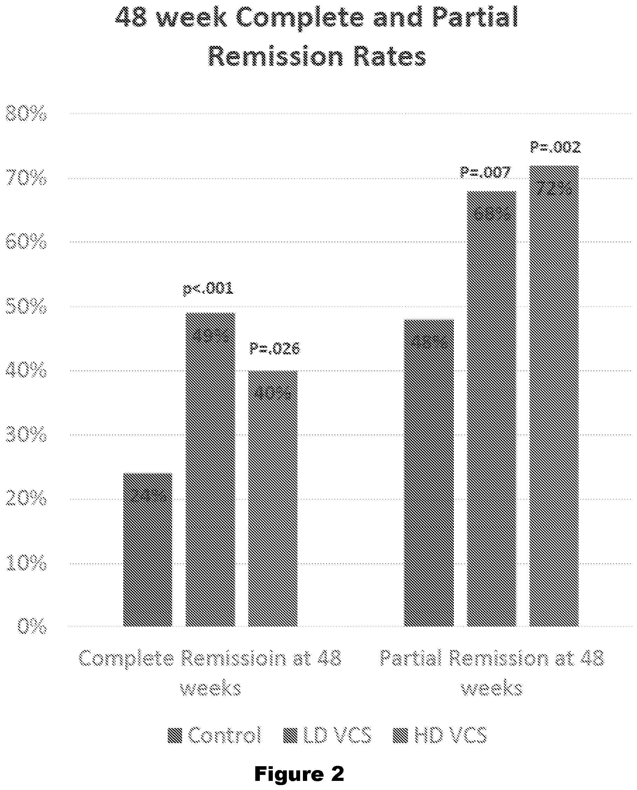

As shown in Figure 2, the low dosage administration achieved better results than administration of voclosporin at higher dosages. After 48 weeks of treatment, 49% of low dosage subjects showed complete remission at 48 weeks, compared to 40% of high dosage subjects. In the same study, but after 24 weeks of treatment, 32.6% of low dosage patients, but only 27% of high dosage patients showed complete remission (CR) compared to 19.3% of controls. At 24 weeks, 70% of low dosage patients and 66% of high dosage patients showed partial remission (PR) compared to 49% of controls.

CR in this example is a composite end-point which includes efficacy, safety and low-dose steroids: UPCR ≤ 0.5mg/mg (confirmed); eGFR >60ml/min/1,73m2 or within 20% of baseline; steroids ≤ 10mg/day; no administration of rescue medication.

PR is a composite end-point that includes safety and efficacy: UPCR reduction of 50% from baseline and no use of rescue medication

To determine the efficacy of the pharmacodynamic protocol wherein dosage is reduced or stopped according to the presence or absence of indicators of the decrease in eGFR experienced as a side effect, these three groups of patients were assessed after 24 weeks and 48 weeks of treatment with respect to whether treatment was altered according to the invention protocol. In all three groups, the patients were evaluated according to the criteria set forth in the exemplary protocol above-*i.e.*, wherein the eGFR of each patient was measured immediately prior to administering the first dose of voclosporin and at a second time point at least a day later and
(i) if the eGFR of said subject decreased by >30% to a value of below 60 mL/min/1.73m² between said first and second time points, stopping the administering of Voclosporin or reducing dosage thereof to said subject;
(ii) if the eGFR of said subject decreased by between 20% to 30% to a value of below 60 ml/min/1.73m² between said first and second time points, administering a reduced dosage of voclosporin to said subject;
(iii) if the eGFR of said subject decreased by <20% between said first and second time points, continuing administering the same predetermined daily dosage of voclosporin to said subject.

The results are shown in Tables 2 and 3 below. Table 2 shows percentages with complete remission (CR) or partial remission (PR) after 24 weeks and Table 3 shows these values after 48 weeks for patients that had no dose reduction and those who did have dose reduction.

**Table 2**

| Patients with a No Dose Reductions (24 weeks): | | | | |
|---|---|---|---|---|
| Group | Patient number (n) | Patients with **no** Dose Reduction n(%) | Patients with **CR** at 24 weeks n(%) | Patients with **PR** at 24 weeks n(%) |
| Placebo | 88 | 77 (87.5) | 14 (18.2) | 36 (46.8) |
| Low Dose | 89 | 50 (56.2) | 15 (30.0) | 34 (68.0) |
| High Dose | 88 | 41 (46.6) | 11 (26.8) | 24 (58.5) |

| Patients with Dose Reductions (pharmacodynamically dosed): | | | | |
|---|---|---|---|---|
| Group | Patient number (n) | Patients with Dose Reduction n(%) | Patients with **CR** at 24 weeks n(%) | Patients with **PR** at 24 weeks n(%) |
| Placebo | 88 | 11 (12.5) | 3 (27.8) | 7 (63.6) |
| Low Dose | 89 | 39 (43.8) | 14 (35.9) | 28 (71.8) |
| High Dose | 88 | 47 (53.4) | 13 (27.7) | 34 (72.3) |

**Table 3**

| Patients with No Dose Reductions (48 weeks): | | | | |
|---|---|---|---|---|
| Group | Patient number (n) | Patients with **no** Dose Reduction n(%) | Patients with **CR** at 48 weeks n(%) | Patients with **PR** at 48 weeks n(%) |
| Placebo | 88 | 74 (84.1) | 18 (24.3) | 38 (51.4) |
| Low Dose | 89 | 43 (48.3) | 20 (46.5) | 26 (60.5) |
| High Dose | 88 | 35 (39.8) | 11 (31.4) | 22 (62.9) |

| Patients with Dose Reductions (pharmacodynamically dosed): | | | | |
|---|---|---|---|---|
| Group | Patient number (n) | Patients with Dose Reduction n(%) | Patients with **CR** at 48 weeks n(%) | Patients with **PR** at 48 weeks n(%) |
| Placebo | 88 | 14 (15.9) | 3 (21.4) | 4 (28.6) |
| Low Dose | 89 | 46 (51.7) | 24 (52.2) | 35 (76.1) |
| High Dose | 88 | 53 (60.2) | 24 (45.3) | 41 (77.4) |

In this study, CR was defined as a composite of UPCR ≤ 0.5 mg/mg; eGFR > 60 mL/min/1.73m² or within 20% of baseline, steroids at ≤ 10 mg/day and no administration of rescue medication. PR is defined as UPCR reduction of 50% from baseline and no use of rescue medication.

As shown in Table 2, 12.5% of patients on placebo, 43.8% of patients on low dose and 53.4% of patients on high dose voclosporin underwent dose reduction during the treatment. The percentage of patients with complete response after 24 weeks was not affected in either dosage groups by the pharmacodynamic dosage and the percentage with partial response was also roughly the same, although with the high dose group, the percentage with partial reduction improved. Table 3 shows similar results at 48 weeks, although a higher percentage of patients were subjected to dose reduction. Again, no drastic effect on the overall response was exhibited.

### Example 2

### Low Dosage Protocol

In the course of clinical studies similar to those in Example 1, it was observed that a substantial portion of subjects showed substantial remission at a dosage reduced almost immediately to 15.8 mg voclosporin administered twice daily (BID). Accordingly, applicants have analyzed these data and have concluded that a dosage protocol providing 15.8 mg or 7.9 mg voclosporin BID is effective with or without the pharmacodynamic aspects of the protocol.

As the capsules contain 7.9mg voclosporin, 1 cap represents 7.9mg voclosporin, 2 caps represent 15.8mg voclosporin and 3 caps represent 23.7mg voclosporin, etc. Substantial numbers of subjects showed complete or partial remission even when the dosage was lowered to 7.9mg voclosporin BID quite early in the treatment and similar results were obtained for administration of 15.8mg BID.

### Example 3

### Predictability based on Early Responses

In the study reported in Example 1, the predictability of outcomes based on markers at early time points were determined. These results are shown in Tables 4-12.

In the study described in Example 1, data were obtained to ascertain whether markers evaluated after various time-points during treatment would predict an ultimate favorable outcome or show that continuing treatment was likely to be futile. This is important because it is undesirable to subject a patient to unnecessary treatment, even if the treatment is relatively safe. These data are shown in the tables below.

Based on these data, the sensitivity and specificity of the evaluation of each marker and their counterparts positive predictive value and negative predictive value were determined with respect to whether the patient would or would not show partial remission (PR) after 48 weeks of the treatment protocol. PR is defined as at least 50% reduction in proteinuria (i.e. UPCR). This would also include subjects who showed complete remission (CR).

Sensitivity is defined as the probability that a subject showing PR at 48 weeks would have shown a favorable result with regard to the marker at the designated early time point. In the tables below, this is the ratio of the number of subjects with favorable marker results (early drop or normalization ) to the total subjects with 48 week PR.

Specificity is defined as the probability that a subject not showing PR at 48 weeks would have shown an unfavorable result with regard to the marker at the designated early time point. In the tables below, this is the ratio of the number of subjects with unfavorable marker results (no early drop or no normalization) to the total subjects with no 48 week PR.

Positive predictive value is defined as the probability that a subject showing a favorable result with respect to the marker at an earlier time point would show PR at 48 weeks. In the tables below, this is the ratio of the number of subjects with favorable marker results who have 48 week PR to those with favorable marker results (early reduction or yes column).

Negative predictive value is defined as the probability that a subject showing an unfavorable result with respect to the marker at an earlier time point would not show PR at 48 weeks. In the tables below, this is the ratio of the number of subjects with unfavorable marker results who have no PR at 48 weeks to the total subjects with unfavorable marker results (no early reduction or no column).

An ideal marker would show a value of 100 for all of these, but this is generally unattainable. As high as possible value is desirable.

Based on the results in these tables, the protocol will be designed to stop treatment at an earlier time point when the parameters set forth above are the most favorable, but at a reasonable time before the 48 week end point. (Obviously the closer to the end point, the more favorable the indicators become, but the advantage to stopping treatment is correspondingly reduced).

**Table 4 - Early 15% reduction in UPCR**

| Table 4 | | Week 48 Partial Remission | | No Week 48 Partial Remission | | Early Reducti on | | | | | |
|---|---|---|---|---|---|---|---|---|---|---|---|
| Week | Treatment Group | Early Drop | No Early Drop | Early Drop | No Early Drop | Yes | No | Sensitivity | Specificity | Positive Predictive Value | Negative Predic-tive Value |
| 2 | Placebo | 27 | 15 | 19 | 25 | 46 | 40 | 64.3 | 36.8 | 58.7 | 62.5 |
| | Voclosporin 23.7mg BID | 45 | 16 | 10 | 14 | 33 | 30 | 73.8 | 58.3 | 81.8 | 46.7 |
| | Voclosporin 39.5mg BID | 39 | 21 | 15 | 9 | 54 | 30 | 65.0 | 37.5 | 72.2 | 30.0 |
| 4 | Placebo | 36 | 5 | 18 | 23 | 34 | 28 | 87.8 | 56.1 | 66.7 | 82.1 |
| | Voclosporin 23.7mg BID | 50 | 9 | 12 | 11 | 62 | 20 | 84.7 | 47.8 | 80.6 | 55.0 |
| | Voclosporin 39.5mg BID | 49 | 12 | 16 | 5 | 65 | 17 | 80.3 | 23.8 | 75.4 | 29.4 |
| 6 | Placebo | 35 | 7 | 22 | 17 | 57 | 24 | 83.3 | 43.6 | 61.4 | 70.8 |
| | Voclosporin 23.7mg BID | 33 | 6 | 12 | 6 | 65 | 12 | 89.8 | 33.3 | 81.5 | 50.0 |
| | Voclosporin 39.5mg BID | 33 | 10 | 16 | 4 | 69 | 14 | 84.1 | 20.0 | 76.8 | 28.6 |
| 8 | Placebo | 38 | 4 | 22 | 18 | 60 | 22 | 90.5 | 45.0 | 63.3 | 81.8 |
| | Voclosporin 23.7mg BID | 33 | 5 | 12 | 7 | 67 | 12 | 91.7 | 36.8 | 82.1 | 58.3 |
| | Voclosporin 39.5mg BID | 36 | 5 | 17 | 4 | 73 | 9 | 91.8 | 19.0 | 76.7 | 44.4 |
| 12 | Placebo | 39 | 3 | 19 | 21 | 58 | 24 | 92.9 | 52.5 | 67.2 | 87.5 |
| | Voclosporin 23.7mg BID | 59 | 2 | 15 | 4 | 74 | 6 | 96.7 | 21.1 | 79.7 | 66.7 |
| | Voclosporin 39.5mg BID | 57 | 5 | 14 | 4 | 71 | 9 | 91.9 | 22.2 | 80.3 | 44.4 |
| 16 | Placebo | 39 | 3 | 19 | 20 | 58 | 23 | 92.9 | 51.3 | 67.2 | 87.0 |
| | Voclosporin 23.7mg BID | 58 | 3 | 13 | 5 | 71 | 8 | 95.1 | 27.8 | 81.7 | 62.5 |
| | Voclosporin 39.5mg BID | 57 | 6 | 15 | 5 | 72 | 11 | 90.5 | 25.0 | 79.2 | 45.5 |
| 20 | Placebo | 38 | 4 | 21 | 16 | 59 | 20 | 90.5 | 43.2 | 64.4 | 80.0 |
| | Voclosporin 23.7mg BID | 60 | 0 | 13 | 3 | 73 | 3 | 100.0 | 18.8 | 82.2 | 100.0 |
| | Voclosporin 39.5mg BID | 58 | 4 | 15 | 5 | 73 | 9 | 93.5 | 25.0 | 79.5 | 55.6 |
| 24 | Placebo | 37 | 3 | 18 | 17 | 33 | 20 | 92.5 | 48.6 | 67.3 | 85.0 |
| | Voclosporin 23.7mg BID | 60 | 0 | 12 | 2 | 72 | 2 | 100.0 | 14.3 | 83.3 | 100.0 |
| | Voclosporin 39.5mg BID | 57 | 5 | 14 | 5 | 71 | 10 | 91.9 | 26.3 | 80.3 | 50.0 |
| 26 | Placebo | 36 | 3 | 16 | 14 | 52 | 17 | 92.3 | 46.7 | 69.2 | 82.4 |
| | Voclosporin 23.7mg BID | 33 | 0 | 9 | 3 | 64 | 3 | 100.0 | 25.0 | 85.9 | 100.0 |
| | Voclosporin 39.5mg BID | 61 | 0 | 12 | 4 | 73 | 4 | 100.0 | 25.0 | 83.6 | 100.0 |
| 36 | Placebo | 41 | 1 | 18 | 13 | 59 | 14 | 97.6 | 41.9 | 69.5 | 92.9 |
| | Voclosporin 23.7mg BID | 60 | 1 | 10 | 3 | 70 | 4 | 98.4 | 23.1 | 85.7 | 75.0 |
| | Voclosporin 39.5mg BID | 62 | 1 | 12 | 6 | 74 | 7 | 98.4 | 33.3 | 83.8 | 85.7 |
| 48 | Placebo | 37 | 1 | 10 | 16 | 47 | 17 | 97.4 | 61.5 | 78.7 | 94.1 |
| | Voclosporin 23.7mg BID | 58 | 0 | 9 | 2 | 67 | 2 | 100.0 | 18.2 | 86.6 | 100.0 |
| | Voclosporin 39.5mg BID | 61 | 0 | 10 | 6 | 71 | 6 | 100.0 | 37.5 | 85.9 | 100.0 |

**Table 5 - Early 20% reduction in UPCR**

| Table 5 | | Week 48 Partial Remission | | No Week 48 Partial Remission | | Early Reduction | | | | | |
|---|---|---|---|---|---|---|---|---|---|---|---|
| Week | Treatment Group | Early Drop | No Early Drop | Early Drop | No Early Drop | Yes | No | Sensitivity | Specificity | Positive Predictive Value | Negative Predic-tive Value |
| 2 | Placebo | 24 | 18 | 15 | 29 | 39 | 47 | 57.1 | 65.9 | 61.5 | 61.7 |
| | Voclosporin 23.7mg BID | 44 | 17 | 8 | 16 | 52 | 33 | 72.1 | 66.7 | 84.6 | 48.5 |
| | Voclosporin 39.5mg BID | 36 | 24 | 14 | 10 | 50 | 34 | 60.0 | 41.7 | 72.0 | 29.4 |
| 4 | Placebo | 32 | 9 | 13 | 28 | 45 | 37 | 78.0 | 68.3 | 71.1 | 75.7 |
| | Voclosporin 23.7mg BID | 49 | 10 | 12 | 11 | 61 | 21 | 83.1 | 47.8 | 80.3 | 52.4 |
| | Voclosporin 39.5mg BID | 47 | 14 | 16 | 5 | 63 | 19 | 77.0 | 23.8 | 74.6 | 26.3 |
| 6 | Placebo | 34 | 8 | 22 | 17 | 36 | 25 | 81.0 | 43.6 | 60.7 | 68.0 |
| | Voclosporin 23.7mg BID | 33 | 6 | 11 | 7 | 64 | 13 | 89.8 | 38.9 | 82.8 | 53.8 |
| | Voclosporin 39.5mg BID | 33 | 10 | 15 | 5 | 68 | 15 | 84.1 | 25.0 | 77.9 | 33.3 |
| 8 | Placebo | 38 | 4 | 19 | 21 | 57 | 25 | 90.5 | 52.5 | 66.7 | 84.0 |
| | Voclosporin 23.7mg BID | 33 | 5 | 11 | 8 | 66 | 13 | 91.7 | | 83.3 | 61.5 |
| | Voclosporin 39.5mg BID | 33 | 6 | 17 | 4 | 72 | 10 | 90.2 | 19.0 | 76.4 | 40.0 |
| 12 | Placebo | 38 | 4 | 19 | 21 | 57 | 25 | 90.5 | 52.5 | 66.7 | 84.0 |
| | Voclosporin 23.7mg BID | 59 | 2 | 14 | 5 | 73 | 7 | 96.7 | 26.3 | 80.8 | 71.4 |
| | Voclosporin 39.5mg BID | 57 | 5 | 14 | 4 | 71 | 9 | 91.9 | 22.2 | 80.3 | 44.4 |
| 16 | Placebo | 39 | 3 | 17 | 22 | 36 | 25 | 92.9 | 56.4 | 69.6 | 88.0 |
| | Voclosporin 23.7mg BID | 58 | 3 | 12 | 6 | 70 | 9 | 95.1 | 33.3 | 82.9 | 66.7 |
| | Voclosporin 39.5mg BID | 57 | 6 | 15 | 5 | 72 | 11 | 90.5 | 25.0 | 79.2 | 45.5 |
| 20 | Placebo | 38 | 4 | 20 | 17 | 58 | 21 | 90.5 | 45.9 | 65.5 | 81.0 |
| | Voclosporin 23.7mg BID | 60 | 0 | 12 | 4 | 72 | 4 | 100.0 | 25.0 | 83.3 | 100.0 |
| | Voclosporin 39.5mg BID | 58 | 4 | 15 | 5 | 73 | 9 | 93.5 | 25.0 | 79.5 | 55.6 |
| 24 | Placebo | 37 | 3 | 16 | 19 | 33 | 22 | 92.5 | 54.3 | 69.8 | 86.4 |
| | Voclosporin 23.7mg BID | 59 | 1 | 11 | 3 | 70 | 4 | 98.3 | 21.4 | 84.3 | 75.0 |
| | Voclosporin 39.5mg BID | 36 | 6 | 14 | 5 | 70 | 11 | 90.3 | 26.3 | 80.0 | 45.5 |
| 26 | Placebo | 36 | 3 | 15 | 15 | 51 | 18 | 92.3 | 50.0 | 70.6 | 83.3 |
| | Voclosporin 23.7mg BID | 33 | 0 | 9 | 3 | 64 | 3 | 100.0 | 25.0 | 85.9 | 100.0 |
| | Voclosporin 39.5mg BID | 61 | 0 | 12 | 4 | 73 | 4 | 100.0 | 25.0 | 83.6 | 100.0 |
| 36 | Placebo | 41 | 1 | 16 | 15 | 57 | 16 | 97.6 | 48.4 | 71.9 | 93.8 |
| | Voclosporin 23.7mg BID | 60 | 1 | 10 | 3 | 70 | 4 | 98.4 | 23.1 | 85.7 | 75.0 |
| | Voclosporin 39.5mg BID | 61 | 2 | 12 | 6 | 73 | 8 | 96.8 | 33.3 | 83.6 | 75.0 |
| 48 | Placebo | 37 | 1 | 9 | 17 | 46 | 18 | 97.4 | 65.4 | 80.4 | 94.4 |
| | Voclosporin 23.7mg BID | 58 | 0 | 9 | 2 | 67 | 2 | 100.0 | 18.2 | 86.6 | 100.0 |
| | Voclosporin 39.5mg BID | 61 | 0 | 10 | 6 | 71 | 6 | 100.0 | 37.5 | 85.9 | 100.0 |

**Table 6 - Early 25% reduction in UPCR**

| Table 6 | | Week 48 Partial Remission | | No Week 48 Partial Remission | | Early Reduction | | | | | |
|---|---|---|---|---|---|---|---|---|---|---|---|
| Week | Treatment Group | Early Drop | No Early Drop | Early Drop | No Early Drop | Yes | No | Sensitivity | Specificity | Positive Predictive Value | Negative Predic-tive Value |
| 2 | Placebo | 18 | 24 | 15 | 29 | 33 | 53 | 42.9 | 65.9 | 54.5 | 54.7 |
| | Voclosporin 23.7mg BID | 39 | 22 | 8 | 16 | 47 | 38 | 63.9 | 66.7 | 83.0 | 42.1 |
| | Voclosporin 39.5mg BID | 33 | 27 | 14 | 10 | 47 | 37 | 55.0 | 41.7 | 70.2 | 27.0 |
| 4 | Placebo | 30 | 11 | 11 | 30 | 41 | 41 | 73.2 | 73.2 | 73.2 | 73.2 |
| | Voclosporin 23.7mg BID | 48 | 11 | 12 | 11 | 60 | 22 | 81.4 | 47.8 | 80.0 | 50.0 |
| | Voclosporin 39.5mg BID | 45 | 16 | 16 | 5 | 61 | 21 | 73.8 | 23.8 | 73.8 | 23.8 |
| 6 | Placebo | 33 | 9 | 20 | 19 | 33 | 28 | 78.6 | 48.7 | 62.3 | 67.9 |
| | Voclosporin 23.7mg BID | 31 | 8 | 10 | 8 | 61 | 16 | 86.4 | 44.4 | 83.6 | 50.0 |
| | Voclosporin 39.5mg BID | 49 | 14 | 15 | 5 | 64 | 19 | 77.8 | 25.0 | 76.6 | 26.3 |
| 8 | Placebo | 33 | 7 | 17 | 23 | 52 | 30 | 83.3 | 57.5 | 67.3 | 76.7 |
| | Voclosporin 23.7mg BID | 34 | 6 | 10 | 9 | 64 | 15 | 90.0 | 47.4 | 84.4 | 60.0 |
| | Voclosporin 39.5mg BID | 33 | 6 | 17 | 4 | 72 | 10 | 90.2 | 19.0 | 76.4 | 40.0 |
| 12 | Placebo | 37 | 5 | 17 | 23 | 34 | 28 | 88.1 | 57.5 | 68.5 | 82.1 |
| | Voclosporin 23.7mg BID | 57 | 4 | 13 | 6 | 70 | 10 | 93.4 | 31.6 | 81.4 | 60.0 |
| | Voclosporin 39.5mg BID | 57 | 5 | 14 | 4 | 71 | 9 | 91.9 | 22.2 | 80.3 | 44.4 |
| 16 | Placebo | 39 | 3 | 17 | 22 | 36 | 25 | 92.9 | 56.4 | 69.6 | 88.0 |
| | Voclosporin 23.7mg BID | 58 | 3 | 11 | 7 | 69 | 10 | 95.1 | 38.9 | 84.1 | 70.0 |
| | Voclosporin 39.5mg BID | 36 | 7 | 14 | 6 | 70 | 13 | 88.9 | 30.0 | 80.0 | 46.2 |
| 20 | Placebo | 38 | 4 | 17 | 20 | 33 | 24 | 90.5 | 54.1 | 69.1 | 83.3 |
| | Voclosporin 23.7mg BID | 59 | 1 | 11 | 5 | 70 | 6 | 98.3 | 31.3 | 84.3 | 83.3 |
| | Voclosporin 39.5mg BID | 58 | 4 | 15 | 5 | 73 | 9 | 93.5 | 25.0 | 79.5 | 55.6 |
| 24 | Placebo | 37 | 3 | 13 | 22 | 50 | 25 | 92.5 | 62.9 | 74.0 | 88.0 |
| | Voclosporin 23.7mg BID | 59 | 1 | 11 | 3 | 70 | 4 | 98.3 | 21.4 | 84.3 | 75.0 |
| | Voclosporin 39.5mg BID | 33 | 7 | 12 | 7 | 67 | 14 | 88.7 | 36.8 | 82.1 | 50.0 |
| 26 | Placebo | 36 | 3 | 14 | 16 | 50 | 19 | 92.3 | 53.3 | 72.0 | 84.2 |
| | Voclosporin 23.7mg BID | 33 | 0 | 8 | 4 | 63 | 4 | 100.0 | 33.3 | 87.3 | 100.0 |
| | Voclosporin 39.5mg BID | 60 | 1 | 12 | 4 | 72 | 5 | 98.4 | 25.0 | 83.3 | 80.0 |
| 36 | Placebo | 41 | 1 | 16 | 15 | 57 | 16 | 97.6 | 48.4 | 71.9 | 93.8 |
| | Voclosporin 23.7mg BID | 60 | 1 | 10 | 3 | 70 | 4 | 98.4 | 23.1 | 85.7 | 75.0 |
| | Voclosporin 39.5mg BID | 61 | 2 | 12 | 6 | 73 | 8 | 96.8 | 33.3 | 83.6 | 75.0 |
| 48 | Placebo | 37 | 1 | 9 | 17 | 46 | 18 | 97.4 | 65.4 | 80.4 | 94.4 |
| | Voclosporin 23.7mg BID | 58 | 0 | 9 | 2 | 67 | 2 | 100.0 | 18.2 | 86.6 | 100.0 |
| | Voclosporin 39.5mg BID | 61 | 0 | 7 | 9 | 68 | 9 | 100.0 | 56.3 | 89.7 | 100.0 |

**Table 7 - Early 30% reduction in UPCR**

| Table 7 | | Week 48 Partial Remission | | No Week 48 Partial Remission | | Early Reduction | | | | | |
|---|---|---|---|---|---|---|---|---|---|---|---|
| Week | Treatment Group | Early Drop | No Early Drop | Early Drop | No Early Drop | Yes | No | Sensitivity | Specificity | Positive Predictive Value | Negative Predic-tive Value |
| 2 | Placebo | 14 | 28 | 13 | 31 | 27 | 59 | 33.3 | 70.5 | 51.9 | 52.5 |
| | Voclosporin 23.7mg BID | 37 | 24 | 6 | 18 | 43 | 42 | 60.7 | 75.0 | 86.0 | 42.9 |
| | Voclosporin 39.5mg BID | 31 | 29 | 11 | 13 | 42 | 42 | 51.7 | 54.2 | 73.8 | 31.0 |
| 4 | Placebo | 27 | 14 | 10 | 31 | 37 | 45 | 65.9 | 75.6 | 73.0 | 68.9 |
| | Voclosporin 23.7mg BID | 47 | 12 | 11 | 12 | 58 | 24 | 79.7 | 52.2 | 81.0 | 50.0 |
| | Voclosporin 39.5mg BID | 45 | 16 | 15 | 6 | 60 | 22 | 73.8 | 28.6 | 75.0 | 27.3 |
| 6 | Placebo | 32 | 10 | 16 | 23 | 48 | 33 | 76.2 | 59.0 | 66.7 | 69.7 |
| | Voclosporin 23.7mg BID | 49 | 10 | 10 | 8 | 59 | 18 | 83.1 | 44.4 | 83.1 | 44.4 |
| | Voclosporin 39.5mg BID | 47 | 16 | 15 | 5 | 62 | 21 | 74.6 | 25.0 | 75.8 | 23.8 |
| 8 | Placebo | 34 | 8 | 15 | 25 | 49 | 33 | 81.0 | 62.5 | 69.4 | 75.8 |
| | Voclosporin 23.7mg BID | 33 | 7 | 10 | 9 | 63 | 16 | 88.3 | 47.4 | 84.1 | 56.3 |
| | Voclosporin 39.5mg BID | 34 | 7 | 17 | 4 | 71 | 11 | 88.5 | 19.0 | 76.1 | 36.4 |
| 12 | Placebo | 36 | 6 | 16 | 24 | 52 | 30 | 85.7 | 60.0 | 69.2 | 80.0 |
| | Voclosporin 23.7mg BID | 36 | 5 | 12 | 7 | 68 | 12 | 91.8 | 36.8 | 82.4 | 58.3 |
| | Voclosporin 39.5mg BID | 33 | 7 | 14 | 4 | 69 | 11 | 88.7 | 22.2 | 79.7 | 36.4 |
| 16 | Placebo | 39 | 3 | 16 | 23 | 33 | 26 | 92.9 | 59.0 | 70.9 | 88.5 |
| | Voclosporin 23.7mg BID | 58 | 3 | 11 | 7 | 69 | 10 | 95.1 | 38.9 | 84.1 | 70.0 |
| | Voclosporin 39.5mg BID | 33 | 8 | 13 | 7 | 68 | 15 | 87.3 | 35.0 | 80.9 | 46.7 |
| 20 | Placebo | 38 | 4 | 16 | 21 | 54 | 25 | 90.5 | 36.8 | 70.4 | 84.0 |
| | Voclosporin 23.7mg BID | 57 | 3 | 9 | 7 | 66 | 10 | 95.0 | 43.8 | 86.4 | 70.0 |
| | Voclosporin 39.5mg BID | 57 | 5 | 15 | 5 | 72 | 10 | 91.9 | 25.0 | 79.2 | 50.0 |
| 24 | Placebo | 37 | 3 | 11 | 24 | 48 | 27 | 92.5 | 68.6 | 77.1 | 88.9 |
| | Voclosporin 23.7mg BID | 59 | 1 | 11 | 3 | 70 | 4 | 98.3 | 21.4 | 84.3 | 75.0 |
| | Voclosporin 39.5mg BID | 33 | 9 | 12 | 7 | 65 | 16 | 85.5 | 36.8 | 81.5 | 43.8 |
| 26 | Placebo | 34 | 5 | 12 | 18 | 46 | 23 | 87.2 | 60.0 | 73.9 | 78.3 |
| | Voclosporin 23.7mg BID | 34 | 1 | 7 | 5 | 61 | 6 | 98.2 | 41.7 | 88.5 | 83.3 |
| | Voclosporin 39.5mg BID | 58 | 3 | 11 | 5 | 69 | 8 | 95.1 | 31.3 | 84.1 | 62.5 |
| 36 | Placebo | 41 | 1 | 14 | 17 | 33 | 18 | 97.6 | 54.8 | 74.5 | 94.4 |
| | Voclosporin 23.7mg BID | 60 | 1 | 10 | 3 | 70 | 4 | 98.4 | 23.1 | 85.7 | 75.0 |
| | Voclosporin 39.5mg BID | 61 | 2 | 11 | 7 | 72 | 9 | 96.8 | 38.9 | 84.7 | 77.8 |
| 48 | Placebo | 37 | 1 | 5 | 21 | 42 | 22 | 97.4 | 80.8 | 88.1 | 95.5 |
| | Voclosporin 23.7mg BID | 58 | 0 | 8 | 3 | 66 | 3 | 100.0 | 27.3 | 87.9 | 100.0 |
| | Voclosporin 39.5mg BID | 61 | 0 | 5 | 11 | 66 | 11 | 100.0 | 68.8 | 92.4 | 100.0 |

**Table 8 - Early 35% reduction in UPCR**

| Table 8 | | Week 48 Partial Remission | | No Week 48 Partial Remission | | Early Reduction | | | | | |
|---|---|---|---|---|---|---|---|---|---|---|---|
| Week | Treatment Group | Early Drop | No Early Drop | Early Drop | No Early Drop | Yes | No | Sensitivity | Specificity | Positive Predictive Value | Negative Predic-tive Value |
| 2 | Placebo | 13 | 29 | 13 | 31 | 26 | 60 | 31.0 | 70.5 | 50.0 | 51.7 |
| | Voclosporin 23.7mg BID | 36 | 25 | 6 | 18 | 42 | 43 | 59.0 | 75.0 | 85.7 | 41.9 |
| | Voclosporin 39.5mg BID | 28 | 32 | 11 | 13 | 39 | 45 | 46.7 | 54.2 | 71.8 | 28.9 |
| 4 | Placebo | 26 | 15 | 9 | 32 | 33 | 47 | 63.4 | 78.0 | 74.3 | 68.1 |
| | Voclosporin 23.7mg BID | 46 | 13 | 11 | 12 | 57 | 25 | 78.0 | 52.2 | 80.7 | 48.0 |
| | Voclosporin 39.5mg BID | 43 | 18 | 13 | 8 | 56 | 26 | 70.5 | 38.1 | 76.8 | 30.8 |
| 6 | Placebo | 30 | 12 | 14 | 25 | 44 | 37 | 71.4 | 64.1 | 68.2 | 67.6 |
| | Voclosporin 23.7mg BID | 47 | 12 | 9 | 9 | 36 | 21 | 79.7 | 50.0 | 83.9 | 42.9 |
| | Voclosporin 39.5mg BID | 47 | 16 | 14 | 6 | 61 | 22 | 74.6 | 30.0 | 77.0 | 27.3 |
| 8 | Placebo | 34 | 8 | 14 | 26 | 48 | 34 | 81.0 | 65.0 | 70.8 | 76.5 |
| | Voclosporin 23.7mg BID | 52 | 8 | 9 | 10 | 61 | 18 | 86.7 | 52.6 | 85.2 | 55.6 |
| | Voclosporin 39.5mg BID | 33 | 8 | 17 | 4 | 70 | 12 | 86.9 | 19.0 | 75.7 | 33.3 |
| 12 | Placebo | 33 | 7 | 14 | 26 | 49 | 33 | 83.3 | 65.0 | 71.4 | 78.8 |
| | Voclosporin 23.7mg BID | 34 | 7 | 10 | 9 | 64 | 16 | 88.5 | 47.4 | 84.4 | 56.3 |
| | Voclosporin 39.5mg BID | 52 | 10 | 14 | 4 | 66 | 14 | 83.9 | 22.2 | 78.8 | 28.6 |
| 16 | Placebo | 39 | 3 | 13 | 26 | 52 | 29 | 92.9 | 66.7 | 75.0 | 89.7 |
| | Voclosporin 23.7mg BID | 57 | 4 | 11 | 7 | 68 | 11 | 93.4 | 38.9 | 83.8 | 63.6 |
| | Voclosporin 39.5mg BID | 33 | 8 | 13 | 7 | 68 | 15 | 87.3 | 35.0 | 80.9 | 46.7 |
| 20 | Placebo | 38 | 4 | 15 | 22 | 33 | 26 | 90.5 | 59.5 | 71.7 | 84.6 |
| | Voclosporin 23.7mg BID | 57 | 3 | 8 | 8 | 65 | 11 | 95.0 | 50.0 | 87.7 | 72.7 |
| | Voclosporin 39.5mg BID | 36 | 6 | 15 | 5 | 71 | 11 | 90.3 | 25.0 | 78.9 | 45.5 |
| 24 | Placebo | 37 | 3 | 10 | 25 | 47 | 28 | 92.5 | 71.4 | 78.7 | 89.3 |
| | Voclosporin 23.7mg BID | 59 | 1 | 8 | 6 | 67 | 7 | 98.3 | 42.9 | 88.1 | 85.7 |
| | Voclosporin 39.5mg BID | 33 | 9 | 12 | 7 | 65 | 16 | 85.5 | 36.8 | 81.5 | 43.8 |
| 26 | Placebo | 34 | 5 | 11 | 19 | 45 | 24 | 87.2 | 63.3 | 75.6 | 79.2 |
| | Voclosporin 23.7mg BID | 52 | 3 | 7 | 5 | 59 | 8 | 94.5 | 41.7 | 88.1 | 62.5 |
| | Voclosporin 39.5mg BID | 33 | 6 | 11 | 5 | 66 | 11 | 90.2 | 31.3 | 83.3 | 45.5 |
| 36 | Placebo | 41 | 1 | 12 | 19 | 53 | 20 | 97.6 | 61.3 | 77.4 | 95.0 |
| | Voclosporin 23.7mg BID | 60 | 1 | 9 | 4 | 69 | 5 | 98.4 | 30.8 | 87.0 | 80.0 |
| | Voclosporin 39.5mg BID | 61 | 2 | 11 | 7 | 72 | 9 | 96.8 | 38.9 | 84.7 | 77.8 |
| 48 | Placebo | 37 | 1 | 3 | 23 | 40 | 24 | 97.4 | 88.5 | 92.5 | 95.8 |
| | Voclosporin 23.7mg BID | 58 | 0 | 7 | 4 | 65 | 4 | 100.0 | 36.4 | 89.2 | 100.0 |
| | Voclosporin 39.5mg BID | 61 | 0 | 4 | 12 | 65 | 12 | 100.0 | 75.0 | 93.8 | 100.0 |

Thus it appears the 8 week or 12 week results, for example, are predictions of ultimate outcome, and it is beneficial to stop treatment if there was no reduction in UPCR by >15% at that time.

For completeness, Tables 9a and 9b show similar results for an 8 week early time point when a 25% UPCR reduction is used as a criterion and the protocol extends for 24 or 48 weeks.

| **Table 9a: Probability of attaining a CR by Proteinuria if UPCR reduction of 25% or greater is achieved at 8 weeks:** | | | | | |
|---|---|---|---|---|---|
| Group | # of patients in treatment arm (number with week 8 assessment) | # of CRs by Proteinuria at 24 weeks | # of patients with UPCR reduction of ≥25% at 8 weeks | #(%) of patients who had a reduction of UPCR of ≥25% who went on to be a CR by UPCR at **24 weeks** | #(%) of patients who had a reduction of UPCR of ≥25% who went on to be a CR by UPCR at **48 weeks** |
| Placebo | 88 (83) | 17 | 52 | 16/52 (30.8) | 18/52 (34.6) |
| Low Dose | 89 (80) | 29 | 64 | 29/64 (45.3) | 40/64 (62.5) |
| High Dose | 88 (84) | 24 | 72 | 24/72 (33.3) | 32/72 (44.4) |

| **Table 9b: Probability of attaining a CR by Proteinuria if UPCR reduction of 25% or greater is not achieved at 8 weeks:** | | | | | |
|---|---|---|---|---|---|
| Group | # of patients in treatment arm (number with week 8 assessment) | # of CRs by Proteinuria at 24 weeks | # of patients **without** UPCR reduction of ≥25% at 8 weeks | #(%) of patients who **did not** have a reduction of UPCR of ≥25% who went on to be a CR by UPCR at **24 weeks** | #(%) of patients who **did not** have a reduction of UPCR of ≥25% who went on to be a CR by UPCR at **48 weeks** |
| Placebo | 88 (83) | 17 | 31 | 1/31 (3.2) | 3/31 (9.7) |
| Low Dose | 89 (80) | 29 | 16 | 0/16 (0.0) | 4/16 (25.0) |
| High Dose | 88 (84) | 24 | 12 | 0/12 (0.0) | 3/12 (25.0) |

Another criterion for effectiveness is normalization of C3 and/or C4 concentration in blood. The normal concentration of C3 is 90 mg/dl or greater and for C4 16 mg/dl or greater. Subjects for the treatment of the invention generally have concentrations below these values. Normalization of C3 is defined as an increase from below 90 mg/dl to above that level or a 25% increase from the baseline (below 90) exhibited by the subject and normalization of C4 is defined as an increase from below 16 mg/dl to above that level or a 25% increase from the baseline (below 16) exhibited by the subject.

Tables 10 - 12 provide similar data for these criteria to the data in Tables 4-9 for UPCR.

**Table 10 - Early C3 normalization**

| Table 10 | | Week 48 Partial Remission | | No Week 48 Partial Remission | | Normal-ization | | | | | |
|---|---|---|---|---|---|---|---|---|---|---|---|
| Week | Treatment Group | Normalization | No Normalization | Normalization | No Normalization | Yes | No | Sensitivity | Specificity | Positive Predictive Value | Negative Predic-tive Value |
| 12 | Placebo | 16 | 24 | 15 | 22 | 31 | 46 | 40.0 | 59.5 | 31.6 | 47.8 |
| | Voclosporin 23.7mg BID | 23 | 34 | 8 | 10 | 31 | 44 | 40.4 | 55.6 | 74.2 | 22.7 |
| | Voclosporin 39.5mg BID | 34 | 28 | 7 | 10 | 41 | 38 | 54.8 | 58.8 | 82.9 | 26.3 |
| 24 | Placebo | 17 | 22 | 16 | 18 | 33 | 40 | 43.6 | 52.9 | 51.5 | 45.0 |
| | Voclosporin 23.7mg BID | 24 | 33 | 6 | 8 | 30 | 41 | 42.1 | 57.1 | 80.0 | 19.5 |
| | Voclosporin 39.5mg BID | 35 | 26 | 6 | 12 | 41 | 38 | 57.4 | 66.7 | 85.4 | 31.6 |
| 48 | Placebo | 15 | 23 | 14 | 13 | 29 | 36 | 39.5 | 48.1 | 51.7 | 36.1 |
| | Voclosporin 23.7mg BID | 25 | 30 | 2 | 8 | 27 | 38 | 45.5 | 80.0 | 92.6 | 21.1 |
| | Voclosporin 39.5mg BID | 34 | 29 | 7 | 9 | 41 | 38 | 54.0 | 56.3 | 82.9 | 23.7 |

**Table 11 - Early C4 normalization**

| Table 11 | | Week 48 Partial Remission | | No Week 48 Partial Remission | | Normal-ization | | | | | |
|---|---|---|---|---|---|---|---|---|---|---|---|
| Week | Treatment Group | Normalization | No Normalization | Normalization | No Normalization | Yes | No | Sensitivity | Specificity | Positive Predictive Value | Negative Predic-tive Value |
| 12 | Placebo | 15 | 25 | 19 | 18 | 34 | 43 | 37.5 | 48.6 | 44.1 | 41.9 |
| | Voclosporin 23.7mg BID | 27 | 29 | 10 | 8 | 37 | 37 | 48.2 | 44.4 | 73.0 | 21.6 |
| | Voclosporin 39.5mg BID | 40 | 22 | 8 | 9 | 48 | 31 | 64.5 | 52.9 | 83.3 | 29.0 |
| 24 | Placebo | 16 | 23 | 18 | 16 | 34 | 39 | 41.0 | 47.1 | 47.1 | 41.0 |
| | Voclosporin 23.7mg BID | 32 | 25 | 8 | 6 | 40 | 31 | 56.1 | 42.9 | 80.0 | 19.4 |
| | Voclosporin 39.5mg BID | 39 | 22 | 5 | 13 | 44 | 33 | 63.9 | 72.2 | 88.6 | 37.1 |
| 48 | Placebo | 16 | 22 | 14 | 13 | 30 | 33 | 42.1 | 48.1 | 33.3 | 37.1 |
| | Voclosporin 23.7mg BID | 29 | 26 | 5 | 5 | 34 | 31 | 52.7 | 50.0 | 85.3 | 16.1 |
| | Voclosporin 39.5mg BID | 36 | 27 | 5 | 11 | 41 | 38 | 57.1 | 68.8 | 87.8 | 28.9 |

**Table 12 - Early C3/C4 normalization**

| Table 12 | | Week 48 Partial Remission | | No Week 48 Partial Remission | | Normal-ization | | | | | |
|---|---|---|---|---|---|---|---|---|---|---|---|
| Wee k | Treatment Group | Normalization | No Normalization | Normalization | No Normalization | Yes | No | Sensitivity | Specificity | Positive Predictive Value | Negative Predic-tive Value |
| 12 | Placebo | 11 | 29 | 14 | 23 | 25 | 52 | 27.5 | 62.2 | 44.0 | 44.2 |
| | Voclosporin 23.7mg BID | 20 | 37 | 6 | 12 | 26 | 49 | 35.1 | 66.7 | 76.9 | 24.5 |
| | Voclosporin 39.5mg BID | 29 | 33 | 7 | 10 | 36 | 43 | 46.8 | 58.8 | 80.6 | 23.3 |
| 24 | Placebo | 12 | 27 | 14 | 20 | 26 | 47 | 30.8 | 58.8 | 46.2 | 42.6 |
| | Voclosporin 23.7mg BID | 23 | 34 | 4 | 10 | 27 | 44 | 40.4 | 71.4 | 85.2 | 22.7 |
| | Voclosporin 39.5mg BID | 26 | 33 | 5 | 13 | 31 | 48 | 42.6 | 72.2 | 83.9 | 27.1 |
| 48 | Placebo | 10 | 28 | 10 | 17 | 20 | 45 | 26.3 | 63.0 | 50.0 | 37.8 |
| | Voclosporin 23.7mg BID | 20 | 33 | 2 | 8 | 22 | 43 | 36.4 | 80.0 | 90.9 | 18.6 |
| | Voclosporin 39.5mg BID | 27 | 36 | 5 | 11 | 32 | 47 | 42.9 | 68.8 | 84.4 | 23.4 |

Tables 13-17 make similar calculations for the combined results of UPCR and C3/C4 at 12 weeks. In these tables, the data from the 12 week determinations in Tables 4-8 are meshed with the data from the 12 week time point in Table 12.

Based on the levels of sensitivity, specificity, positive predictive value and negative predicted value shown, decision to stop or continue treatment is made.

**Table 13 - Early 15% reduction in UPCR or C3/C4 normalization**

| Table 13 | | Week 48 Partial Remission | | No Week 48 Partial Remission | | Early Drop or Normal | | | | | |
|---|---|---|---|---|---|---|---|---|---|---|---|
| Week | Treatment Group | Early Drop or Normal | No Early Drop or Normal | Early Drop or Normal | No Early Drop or Normal | Yes | No | Sensitivity | Specificity | Positive Predictive Value | Negative Predic-tive Value |
| 12 | Placebo | 39 | 3 | 26 | 12 | 65 | 15 | 92.9 | 31.6 | 60.0 | 80.0 |
| | Voclosporin 23.7mg BID | 60 | 1 | 18 | 1 | 78 | 2 | 98.4 | 5.3 | 76.9 | 50.0 |
| | Voclosporin 39.5mg BID | 60 | 3 | 16 | 3 | 76 | 6 | 95.2 | 15.8 | 78.9 | 50.0 |
| 24 | Placebo | 39 | 2 | 27 | 8 | 66 | 10 | 95.1 | 22.9 | 59.1 | 80.0 |
| | Voclosporin 23.7mg BID | 61 | 0 | 13 | 1 | 74 | 1 | 100.0 | 7.1 | | 82.4 100.0 |
| | Voclosporin 39.5mg BID | 57 | 5 | 15 | 4 | 72 | 9 | 91.9 | 21.1 | 79.2 | 44.4 |
| 48 | Placebo | 37 | 1 | 18 | 9 | 33 | 10 | 97.4 | 33.3 | 67.3 | 90.0 |
| | Voclosporin 23.7mg BID | 58 | 0 | 10 | 1 | 68 | 1 | 100.0 | 9.1 | 85.3 | 100.0 |
| | Voclosporin 39.5mg BID | 62 | 0 | 12 | 4 | 74 | 4 | 100.0 | 25.0 | 83.8 | 100.0 |

**Table 14 - Early 20% reduction in UPCR or C3/C4 normalization**

| Table 14 | | Week 48 Partial Remission | | No Week 48 Partial Remission | | Early Drop or Normal | | | | | |
|---|---|---|---|---|---|---|---|---|---|---|---|
| Week | Treatment Group | Early Drop or Normal | No Early Drop or Normal | Early Drop or Normal | No Early Drop or Normal | Yes | No | Sensitivity | Specificity | Positive Predictive Value | Negative Predic-tive Value |
| 12 | Placebo | 38 | 4 | 26 | 12 | 64 | 16 | 90.5 | 31.6 | 59.4 | 75.0 |
| | Voclosporin 23.7mg BID | 60 | 1 | 17 | 2 | 77 | 3 | 98.4 | 10.5 | 77.9 | 66.7 |
| | Voclosporin 39.5mg BID | 60 | 3 | 16 | 3 | 76 | 6 | 95.2 | 15.8 | 78.9 | 50.0 |
| 24 | Placebo | 39 | 2 | 25 | 10 | 64 | 12 | 95.1 | 28.6 | 60.9 | 83.3 |
| | Voclosporin 23.7mg BID | 61 | 0 | 13 | 1 | 74 | 1 | 100.0 | 7.1 | | 82.4 100.0 |
| | Voclosporin 39.5mg BID | 57 | 5 | 15 | 4 | 72 | 9 | 91.9 | 21.1 | 79.2 | 44.4 |
| 48 | Placebo | 37 | 1 | 17 | 10 | 54 | 11 | 97.4 | 37.0 | 68.5 | 90.9 |
| | Voclosporin 23.7mg BID | 58 | 0 | 10 | 1 | 68 | 1 | 100.0 | 9.1 | 85.3 | 100.0 |
| | Voclosporin 39.5mg BID | 62 | 0 | 12 | 4 | 74 | 4 | 100.0 | 25.0 | 83.8 | 100.0 |

**Table 15 - Early 25% reduction in UPCR or C3/C4 normalization**

| Table 15 | | Week 48 Partial Remission | | No Week 48 Partial Remission | | Early Drop or Normal | | | | | |
|---|---|---|---|---|---|---|---|---|---|---|---|
| Week | Treatment Group | Early Drop or Normal | No Early Drop or Normal | Early Drop or Normal | No Early Drop or Normal | Yes | No | Sensitivity | Specificity | Positive Predictive Value | Negative Predic-tive Value |
| 12 | Placebo | 38 | 4 | 24 | 14 | 62 | 18 | 90.5 | 36.8 | 61.3 | 77.8 |
| | Voclosporin 23.7mg BID | 58 | 2 | 16 | 3 | 74 | 5 | 96.7 | 15.8 | 78.4 | 60.0 |
| | Voclosporin 39.5mg BID | 60 | 3 | 16 | 3 | 76 | 6 | 95.2 | 15.8 | 78.9 | 50.0 |
| 24 | Placebo | 39 | 2 | 23 | 12 | 62 | 14 | 95.1 | 34.3 | 62.9 | 85.7 |
| | Voclosporin 23.7mg BID | 61 | 0 | 13 | 1 | 74 | 1 | 100.0 | 7.1 | | 82.4 100.0 |
| | Voclosporin 39.5mg BID | 57 | 5 | 14 | 5 | 71 | 10 | 91.9 | 26.3 | 80.3 | 50.0 |
| 48 | Placebo | 37 | 1 | 17 | 10 | 34 | 11 | 97.4 | 37.0 | 68.5 | 90.9 |
| | Voclosporin 23.7mg BID | 58 | 0 | 10 | 1 | 68 | 1 | 100.0 | 9.1 | 85.3 | 100.0 |
| | Voclosporin 39.5mg BID | 62 | 0 | 10 | 6 | 72 | 6 | 100.0 | 37.5 | 86.1 | 100.0 |

**Table 16 - Early 30% reduction in UPCR or C3/C4 normalization**

| Table 16 | | Week 48 Partial Remission | | No Week 48 Partial Remission | | Early Drop or Normal | | | | | |
|---|---|---|---|---|---|---|---|---|---|---|---|
| Week | Treatment Group | Early Drop or Normal | No Early Drop or Normal | Early Drop or Normal | No Early Drop or Normal | Yes | No | Sensitivity | Specificity | Positive Predictive Value | Negative Predic-tive Value |
| 12 | Placebo | 37 | 5 | 24 | 14 | 61 | 19 | 88.1 | 36.8 | 60.7 | 73.7 |
| | Voclosporin 23.7mg BID | 58 | 2 | 16 | 3 | 74 | 5 | 96.7 | 15.8 | 78.4 | 60.0 |
| | Voclosporin 39.5mg BID | 59 | 4 | 16 | 3 | 73 | 7 | 93.7 | 15.8 | 78.7 | 42.9 |
| 24 | Placebo | 39 | 2 | 22 | 13 | 61 | 15 | 95.1 | 37.1 | 63.9 | 86.7 |
| | Voclosporin 23.7mg BID | 61 | 0 | 13 | 1 | 74 | 1 | 100.0 | 7.1 | 82.4 | 100.0 |
| | Voclosporin 39.5mg BID | 57 | 5 | 14 | 5 | 71 | 10 | 91.9 | 26.3 | 80.3 | 50.0 |
| 48 | Placebo | 37 | 1 | 13 | 14 | 50 | 15 | 97.4 | 51.9 | 74.0 | 93.3 |
| | Voclosporin 23.7mg BID | 58 | 0 | 9 | 2 | 67 | 2 | 100.0 | 18.2 | 86.6 | 100.0 |
| | Voclosporin 39.5mg BID | 62 | 0 | 8 | 8 | 70 | 8 | 100.0 | 50.0 | 88.6 | 100.0 |

**Table 17 - Early 35% reduction in UPCR or C3/C4 normalization**

| Table 17 | | Week 48 Partial Remission | | No Week 48 Partial Remission | | Early Drop or Normal | | | | | |
|---|---|---|---|---|---|---|---|---|---|---|---|
| Week | Treatment Group | Early Drop or Normal | No Early Drop or Normal | Early Drop or Normal | No Early Drop or Normal | Yes | No | Sensitivity | Specificity | Positive Predictive Value | Negative Predic-tive Value |
| 12 | Placebo | 36 | 6 | 23 | 15 | 59 | 21 | 85.7 | 39.5 | 61.0 | 71.4 |
| | Voclosporin 23.7mg BID | 56 | 4 | 14 | 5 | 70 | 9 | 93.3 | 26.3 | 80.0 | 55.6 |
| | Voclosporin 39.5mg BID | 57 | 6 | 16 | 3 | 73 | 9 | 90.5 | 15.8 | 78.1 | 33.3 |
| 24 | Placebo | 39 | 2 | 21 | 14 | 60 | 16 | 95.1 | 40.0 | 65.0 | 87.5 |
| | Voclosporin 23.7mg BID | 61 | 0 | 10 | 4 | 71 | 4 | 100.0 | 28.6 | 85.9 | 100.0 |
| | Voclosporin 39.5mg BID | 57 | 5 | 14 | 5 | 71 | 10 | 91.9 | 26.3 | 80.3 | 50.0 |
| 48 | Placebo | 37 | 1 | 12 | 15 | 49 | 16 | 97.4 | 55.6 | 75.5 | 93.8 |
| | Voclosporin 23.7mg BID | 58 | 0 | 8 | 3 | 66 | 3 | 100.0 | 27.3 | 87.9 | 100.0 |
| | Voclosporin 39.5mg BID | 62 | 0 | 7 | 9 | 69 | 9 | 100.0 | 56.3 | 89.9 | 100.0 |

### Example 4

### Low Dose Corticosteroid

Applicants have also found that the dosage of corticosteroid can effectively be reduced as compared to "standard of care" as shown in Tables 8 and 9, and can be reduced further to 4mg per day or less.

**Table 18**

| Standard of Care Dosing Schedule for IV methylprednisolone and daily oral prednisone: | | |
|---|---|---|
| Time | Patients < 45 kg (daily dosage) | Patients >_ 45 kg (daily dosage) |
| | | |
| Days 1-3 | 0.5 g IV methylprednisolone | 1 g IV methylprednisolone |
| | | |
| Days 3-112 | 1 mg/kg tapered down | 1 mg/kg (maximum 80 mg) tapered down |

**Table 19**

| Lowered Dosing Schedule for IV methylprednisolone and daily oral prednisone: | | |
|---|---|---|
| Time | Patients < 45 kg (daily dosage) | Patients ≥ 45 kg (daily dosage) |
| | | |
| Days 1-2 | 0.25 g IV methylprednisolone | 0.5 g IV methylprednisolone |
| | | |
| Days 3-13 | 20 mg oral prednisone | 25 mg oral prednisone |
| Week 3 | 15 mg oral prednisone | 20 mg oral prednisone |
| Week 4 | 10 mg oral prednisone | 15 mg oral prednisone |
| Week 6 | 10 mg oral prednisone | 10 mg oral prednisone |
| Week 8 | 5 mg oral prednisone | 5 mg oral prednisone |
| Week 12 | 5 mg oral prednisone | 5 mg oral prednisone |
| Week 16 | 2.5 mg oral prednisone | 2.5 mg oral prednisone |

## Claims

1. Voclosporin for use in a method to treat a proteinuric kidney disease of a subject diagnosed with said disease, with a predetermined daily dosage of effective amounts of voclosporin is to be administered over a projected treatment period of at least 24 weeks, wherein:
(a)the estimated Glomerular Filtration Rate (eGFR) of said subject is assessed at at least a first time point and a second time point on different days of said treatment period, both said time points occurring prior to the end of the treatment period and
(b)
(i) if the eGFR of said subject is decreased, between said first and second time points, by more than a target % in the range of 20-45%, to below a predetermined value in the range of 50-90 ml/min/1.73m², the daily dosage is reduced by increment(s) of 7.9mg BID;
(ii) if the eGFR of said subject is decreased, between said first and second time points, by less than said target %, administration of the same predetermined daily dosage of voclosporin to said subject is continued.

2. Voclosporin for use according to claim 1, wherein the daily dosage is reduced to 15.8 mg BID in step (b) (i) of claim 1.

3. Voclosporin for use according to claim 1, wherein the daily dosage is reduced to 7.9 mg BID in step (b) (i) of claim 1.

4. Voclosporin for use according to any of claims 1-3, wherein the first time point is immediately preceding initiating said protocol.

5. Voclosporin for use according to any of claims 1-4, wherein the predetermined value is approximately 60ml/min/1.73m².

6. Voclosporin for use according to claim 5, wherein the daily dosage is reduced to zero in step (b) (i) of claim 1.

7. Voclosporin for use according to any of claims 1-6 wherein the target % is approximately 30%.

8. Voclosporin for use according to any one of claims 1-7, wherein said subject has been identified as appropriate for said use prior to the use being conducted on said subject, wherein:
(a) the urine protein creatinine ratio (UPCR) of said subject has been determined being >1mg/mg as measured by first morning void or 24 hour urine; and
(b) said subject has been determined as having an eGFR as measured by Chronic Kidney Disease Epidemiology Collaboration equation (CKD-EPI) of >45 ml/min/1.73m²,
wherein if the conditions of (a) and (b) are met, said subject has been identified as appropriate for said use.

9. Voclosporin for use according to any of claims 1-8, wherein said predetermined daily dosage is 39.5mg voclosporin BID or 31.6mg voclosporin BID or 23.7mg voclosporin BID or 15.8mg voclosporin BID or 7.9mg voclosporin BID.

10. Voclosporin for use according to any of claims 1-9, wherein said predetermined daily dosage is 23.7mg voclosporin BID.

11. Voclosporin for use according to any of claims 1-10, wherein said method further includes evaluating said subject for renal function at a time point after the end of said treatment period by assessing eGFR.

12. Voclosporin for use according to claim 11, wherein said subject is further evaluated for efficacy by assessing protein/creatinine ratio (UPCR) at a time point after the end of said treatment period.

13. Voclosporin for use according to any of claims 1-12, wherein an effective amount of mycophenolate mofetil (MMF) is further administered to said subject.

14. Voclosporin for use according to any of claims 1-13, wherein an effective amount of a corticosteroid is administered to said subject.

15. Voclosporin for use according to any of claims 1-14, wherein said treatment period is at least 48 weeks.

16. Voclosporin for use according to any of claims 1-15, wherein the eGFR of said subject is determined at a third time point and if the eGFR is determined at said third time point to differ from the eGFR determined at said first time point by less than said target %, administration of said predetermined daily dosage of voclosporin is resumed.

17. Voclosporin for use according to claim 16, wherein the target % is 20-45%.

18. Voclosporin for use according to claim 17, wherein the target % is approximately 30%.

19. Voclosporin for use according to any of claim 1-18, wherein the proteinuric kidney disease is lupus nephritis.

## Patentansprüche

1. Voclosporin zur Verwendung in einem Verfahren zum Behandeln einer proteinurischen Nierenerkrankung eines Subjekts, diagnostiziert mit der Erkrankung, wobei eine vorbestimmte Tagesdosis wirksamer Mengen von Voclosporin über einen geplanten Behandlungszeitraum von wenigstens 24 Wochen verabreicht werden soll, wobei:
(a) die geschätzte glomeruläre Filtrationsrate (eGFR) des Subjekts zu wenigstens einem ersten Zeitpunkt und einem zweiten Zeitpunkt an verschiedenen Tagen des Behandlungszeitraums festgestellt wird, wobei beide Zeitpunkte vor dem Ende des Behandlungszeitraums eintreten und
(b)
(i) wenn die eGFR des Subjekts zwischen dem ersten und dem zweiten Zeitpunkt um mehr als einen Zielprozentsatz in dem Bereich von 20 bis 45 % auf unter einen vorbestimmten Wert in dem Bereich von 50 bis 90 ml/min/1.73m² gefallen ist, die Tagesdosis um Inkrement(e) von 7,9 mg BID reduziert wird;
(ii) wenn die eGFR des Subjekts zwischen dem ersten und dem zweiten Zeitpunkt um weniger als den Zielprozentsatz gesunken ist, Verabreichung der gleichen vorbestimmten Tagesdosis von Voclosporin an das Subjekt fortgesetzt wird.

2. Voclosporin zur Verwendung nach Anspruch 1, wobei die tägliche Dosierung auf 15,8 mg BID in Schritt (b) (i) von Anspruch 1 reduziert wird.

3. Voclosporin zur Verwendung nach Anspruch 1, wobei die Tagesdosis auf 7,9 mg BID in Schritt (b) (i) von Anspruch 1 reduziert wird.

4. Voclosporin zur Verwendung nach einem der Ansprüche 1 - 3, wobei der erste Zeitpunkt unmittelbar vor Initiieren des Protokolls ist.

5. Voclosporin zur Verwendung nach einem der Ansprüche 1 - 4, wobei der vorbestimmte Wert ungefähr 60 ml/min/1,73m² ist.

6. Voclosporin zur Verwendung nach Anspruch 5, wobei die Tagesdosis auf Null in Schritt (b) (i) von Anspruch 1 reduziert wird.

7. Voclosporin zur Verwendung nach einem der Ansprüche 1 - 6, wobei der Zielprozentsatz ungefähr 30 % ist.

8. Voclosporin zur Verwendung nach einem der Ansprüche 1 - 7, wobei das Subjekt als geeignet für die Verwendung identifiziert wurde, bevor die Verwendung an dem Subjekt durchgeführt wird, wobei:
(a) das Urin-Protein-Kreatinin-Verhältnis (UPCR) des Subjekts mit ≥ 1mg/mg, wie gemessen durch erste morgendliche Entleerung oder 24-Stunden-Urin, bestimmt wurde; und
(b) das Subjekt als habend eine eGFR, wie gemessen durch die Gleichung der Chronic Kidney Disease Epidemiology Collaboration (CKD-EPI), von ≥45 ml/min/1,73m², bestimmt wurde,
wobei, wenn die Bedingungen von (a) und (b) erfüllt sind, das Subjekt als geeignet für die Verwendung identifiziert wurde.

9. Voclosporin zur Verwendung nach einem der Ansprüche 1 - 8, wobei die vorbestimmte Tagesdosis 39,5 mg Voclosporin BID oder 31,6 mg Voclosporin BID oder 23,7 mg Voclosporin BID oder 15,8 mg Voclosporin BID oder 7,9 mg Voclosporin BID ist.

10. Voclosporin zur Verwendung nach einem der Ansprüche 1 - 9, wobei die vorbestimmte Tagesdosis 23,7 mg Voclosporin BID ist.

11. Voclosporin zur Verwendung nach einem der Ansprüche 1 - 10, wobei das Verfahren ferner Bewerten der Nierenfunktion des Subjekts zu einem Zeitpunkt nach dem Ende des Behandlungszeitraums durch Feststellen von eGFR einschließt.

12. Voclosporin zur Verwendung nach Anspruch 11, wobei das Subjekt ferner auf Wirksamkeit durch Feststellen von Protein/Kreatinin-Verhältnis (UPCR) zu einem Zeitpunkt nach dem Ende der Behandlungsperiode bewertet wird.

13. Voclosporin zur Verwendung nach einem der Ansprüche 1 - 12, wobei dem Subjekt ferner eine wirksame Menge von Mycophenolatmofetil (MMF) verabreicht wird.

14. Voclosporin zur Verwendung nach einem der Patentansprüche 1 - 13, wobei dem Subjekt eine effektive Menge eines Kortikosteroids verabreicht wird.

15. Voclosporin zur Verwendung nach einem der Ansprüche 1-14, wobei die Behandlungsdauer wenigstens 48 Wochen ist.

16. Voclosporin zur Verwendung nach einem der Ansprüche 1 - 15, wobei die eGFR des Subjekts an einem dritten Zeitpunkt bestimmt wird und, wenn die eGFR zu dem dritten Zeitpunkt bestimmt wird, sich von der eGFR, bestimmt an dem ersten Zeitpunkt, um weniger als die Ziel-% zu unterscheiden, Verabreichung der vorbestimmten Tagesdosis von Voclosporin wieder aufgenommen wird.

17. Voclosporin zur Verwendung nach Anspruch 16, wobei der Zielprozentsatz 20 - 45% ist.

18. Voclosporin zur Verwendung nach Anspruch 17, wobei der Zielprozentsatz ungefähr 30 % ist.

19. Voclosporin zur Verwendung nach einem der Ansprüche 1 - 18, wobei die proteinurische Nierenerkrankung Lupus-Nephritis ist.

## Revendications

1. Voclosporine pour son utilisation dans une méthode de traitement d'une maladie rénale protéinurique d'un sujet chez qui ladite maladie a été diagnostiquée, avec une dose quotidienne prédéterminée de quantités efficaces de voclosporine qui doit être administrée au cours d'une période de traitement prévue d'au moins 24 semaines, dans laquelle :
(a) le débit de filtration glomérulaire estimé (DFGe) dudit sujet est évalué au moins à un premier point temporel et un deuxième point temporel lors de différents jours de ladite période de traitement, lesdits deux points temporels survenant avant la fin de la période de traitement et
(b)
(i) si le DFGe dudit sujet est réduit, entre lesdits premier et deuxième points temporels, de plus d'un pourcentage cible dans la plage de 20 à 45 %, à moins d'une valeur prédéterminée dans la plage de 50 à 90 ml/min/1,73 m², la dose quotidienne est réduite par incrément(s) de 7,9 mg BID ;
(ii) si le DFGe dudit sujet est réduit, entre lesdits premier et deuxième points temporels, de moins dudit pourcentage cible, l'administration de la même dose quotidienne prédéterminée de voclosporine audit sujet se poursuit.

2. Voclosporine pour son utilisation selon la revendication 1, dans laquelle la dose quotidienne est réduite à 15,8 mg BID à l'étape (b) (i) de la revendication 1.

3. Voclosporine pour son utilisation selon la revendication 1, dans laquelle la dose quotidienne est réduite à 7,9 mg BID à l'étape (b) (i) de la revendication 1.

4. Voclosporine pour son utilisation selon l'une quelconque des revendications 1 à 3, dans laquelle le premier point temporel précède immédiatement l'initiation dudit protocole.

5. Voclosporine pour son utilisation selon l'une quelconque des revendications 1 à 4, dans laquelle la valeur prédéterminée est d'approximativement 60 ml/min/1,73 m².

6. Voclosporine pour son utilisation selon la revendication 5, dans laquelle la dose quotidienne est réduite à zéro à l'étape (b) (i) de la revendication 1.

7. Voclosporine pour son utilisation selon l'une quelconque des revendications 1 à 6, dans laquelle le pourcentage cible est d'approximativement 30 %.

8. Voclosporine pour son utilisation selon l'une quelconque des revendications 1 à 7, dans laquelle ledit sujet a été identifié comme approprié pour ladite utilisation avant que l'utilisation ne soit réalisée sur ledit sujet, dans laquelle :
(a) le rapport protéinurie/créatininurie (RPCU) dudit sujet a été déterminé comme étant ≥ 1 mg/mg tel que mesuré par la première miction matinale ou un recueil des urines sur 24 heures ; et
(b) ledit sujet a été déterminé comme ayant un DFGe tel que mesuré par l'équation CKD-EPI (Chronic Kidney Disease Epidemiology Collaboration) ≥ 45 ml/min/1,73 m²,
dans laquelle si les conditions de (a) et (b) sont satisfaites, ledit sujet a été identifié comme approprié pour ladite utilisation.

9. Voclosporine pour son utilisation selon l'une quelconque des revendications 1 à 8, dans laquelle ladite dose quotidienne prédéterminée est de 39,5 mg de voclosporine BID ou 31,6 mg de voclosporine BID ou 23,7 mg de voclosporine BID ou 15,8 mg de voclosporine BID ou 7,9 mg de voclosporine BID.

10. Voclosporine pour son utilisation selon l'une quelconque des revendications 1 à 9, dans laquelle ladite dose quotidienne prédéterminée est de 23,7 mg de voclosporine BID.

11. Voclosporine pour son utilisation selon l'une quelconque des revendications 1 à 10, dans laquelle ladite méthode comporte en outre l'appréciation de la fonction rénale dudit sujet à un point temporel après la fin de ladite période de traitement par évaluation du DFGe.

12. Voclosporine pour son utilisation selon la revendication 11, dans laquelle l'efficacité est en outre appréciée chez ledit sujet par estimation du rapport protéine/créatinine (RPC) à un point temporel après la fin de ladite période de traitement.

13. Voclosporine pour son utilisation selon l'une quelconque des revendications 1 à 12, dans laquelle une quantité efficace de mycophénolate mofétil (MMF) est en outre administrée audit sujet.

14. Voclosporine pour son utilisation selon l'une quelconque des revendications 1 à 13, dans laquelle une quantité efficace d'un corticoïde est administrée audit sujet.

15. Voclosporine pour son utilisation selon l'une quelconque des revendications 1 à 14, dans laquelle ladite période de traitement est d'au moins 48 semaines.

16. Voclosporine pour son utilisation selon l'une quelconque des revendications 1 à 15, dans laquelle le DFGe dudit sujet est déterminé à un troisième point temporel et si le DFGe est déterminé audit troisième point temporel comme étant différent du DFGe déterminé audit premier point temporel de moins dudit pourcentage cible, l'administration de ladite dose quotidienne prédéterminée de voclosporine est reprise.

17. Voclosporine pour son utilisation selon la revendication 16, dans laquelle le pourcentage cible est de 20 à 45 %.

18. Voclosporine pour son utilisation selon la revendication 17, dans laquelle le pourcentage cible est d'approximativement 30 %.

19. Voclosporine pour son utilisation selon l'une quelconque des revendications 1 à 18, dans laquelle la maladie rénale protéinurique est une néphropathie lupique.
